Europäisches Patentamt

European Patent Office

Office européen des brevets

(11) Veröffentlichungsnummer: **0 319 809**
**A2**

## EUROPÄISCHE PATENTANMELDUNG

(21) Anmeldenummer: 88119743.8

(51) Int. Cl.⁴: **C07C 57/13 , C07C 51/567**

(22) Anmeldetag: 26.11.88

(30) Priorität: 11.12.87 AT 3521/87

(43) Veröffentlichungstag der Anmeldung:
14.06.89 Patentblatt 89/24

(84) Benannte Vertragsstaaten:
DE FR GB IT

(71) Anmelder: **Vianova Kunstharz Aktiengesellschaft**

**A-8402 Werndorf(AT)**

(72) Erfinder: **Kriessmann, Ingo, Dr.**
**Hilmteichstrasse 75**
**A-8010 Graz(AT)**
Erfinder: **Kolmayr, Anton, Dr.**
**Pfalzgrafenweg 31**
**A-8020 Graz(AT)**

(54) Verfahren zur Herstellung von Alkenylbernsteinsäureanhydriden mit verbesserter Eigenfarbe.

(57) Die Erfindung betrifft ein Verfahren zur Herstellung von Alkenylbernsteinsäureanhydriden mit verbesserter Eigenfarbe durch die Anlagerungsreaktion von Maleinsäureanhydrid an die entsprechenden Olefine in Gegenwart von Kombinationen der Oxalsäure und phenolischen Antioxidantien.

EP 0 319 809 A2

## Verfahren zur Herstellung von Alkenylbernsteinsäureanhydriden mit verbesserter Eigenfarbe

Die Erfindung betrifft ein Verfahren zur Herstellung von Alkenylbernsteinsäureanhydriden mit verbesserter Eigenfarbe durch die Anlagerungsreaktion von Maleinsäureanhydrid an die entsprechenden Olefine in Gegenwart von Kombinationen der Oxalsäure und phenolischen Antioxidantien.

Alkenylbernsteinsäureanhydride und deren Derivate werden als Additive für Öle und Schmierstoffe, als Härter für Epoxidharze, als Weichmacher und als Tenside in der Waschmittelindustrie eingesetzt.

Die Herstellung von Alkenylbernsteinsäureanhydriden erfolgt üblicherweise durch eine Anlagerungsreaktion von Maleinsäureanhydrid an die entsprechenden Olefine bei Reaktionstemperaturen von 160 - 230° C und Reaktionszeiten bis zu 24 Stunden, wobei die Olefine im Überschuß eingesetzt werden.

Unter diesen Bedingungen besteht die Tendenz zur Bildung von Eigenpolymerisaten, besonders seitens der Olefine, was zu dunkel gefärbten und damit nur beschränkt verwendbaren Reaktionsprodukten führen kann.

Es hat daher an Versuchen nicht gefehlt, die Eigenfarbe der Endprodukte zu verbessern.

So werden in der GB - 1.337.724 Zusätze von organischen Phosphorverbindungen und organischer Antioxidantien beschrieben, deren Wirkung gemäß GB - 1.396.097 durch die Anwesenheit von Metallionen noch verstärkt werden kann. Auf diese Kombinationen verweist auch die DE-OS-22 55 571.

Im Rahmen der zunehmend strengen behördlichen Vorschriften, wie sie speziell der Waschmittelindustrie auferlegt werden, ist es wünschenswert, möglichst ohne Zusätze von Phosphorverbindungen in der Eigenfarbe zufriedenstellende Alkenylbernsteinsäureanhydride herzustellen.

Es wurde nun gefunden, daß die Eigenfarbe von Alkenylbernsteinsäureanhydriden wesentlich verbessert werden kann, wenn die Additionsreaktion von $C_8$-$C_{13}$-Monoolefinen an Maleinsäureanhydrid in Gegenwart von Oxalsäure durchgeführt wird, wobei gleichzeitig die erforderliche Menge an phenolischen Antioxydantien verringert werden kann.

Die Erfindung betrifft demgemäß ein Verfahren zur Herstellung von Alkenylbernsteinsäureanhydriden mit verbesserter Eigenfarbe durch Umsetzung von Maleinsäureanhydrid mit einem molaren Überschuß an $C_8$-$C_{13}$-Monoolefinen, in Gegenwart eines phenolischen Antioxidans, welches dadurch gekennzeichnet ist, daß die Additionsreaktion in Gegenwart von, bezogen auf den gesamten Reaktionsansatz, 0,04 bis 0,15 Gew.-%, vorzugsweise 0,05 bis 0,12 Gew.-%, Oxalsäure und von 0,04 bis 0,12 Gew.-% eines phenolischen Antioxidans durchgeführt wird, mit der Maßgabe, daß die Oxalsäure in zwei Anteilen von mindestens 30 Gew.-%, bevorzugt in Anteilen von je 50 Gew.-%, bezogen auf die gesamte Menge an Oxalsäure, zu Beginn der Additionsreaktion und vor der Destillationsphase zugegeben wird.

Durch die Anwendung des erfindungsgemäßen Verfahrens kann gegenüber den heute in der Praxis üblichen Verfahren unter Verwendung von Triphenylphosphit und phenolischen Antioxidantien eine wesentliche Verbesserung der Farbe des Endproduktes erzielt werden. Zusätzlich erreicht man eine Reduzierung des phenolischen Antioxidans um etwa die Hälfte.

Die Herstellung von Alkenylbernsteinsäureanhydriden durch Umsetzung von Maleinsäureanhydrid mit einem molaren Überschuß an $C_8$- $C_{13}$-Monoolefinen wird bei Reaktionstemperaturen von 180 - 210° C und Reaktionszeiten bis zu 24 Stunden unter nachfolgender Entfernung nicht umgesetzter Monoolefine durch Vakuumdestillation durchgeführt.

Als Olefine können handelsübliche Olefine eingesetzt werden. Bevorzugt verwendet werden Olefine mit einer endständigen Doppelbindung, wie Octen-1, Undecen-1 und Tetradecen-1.

Die Oxalsäure wird in Mengen von, bezogen auf den gesamten Reaktionsansatz, 0,04 bis 0,15 Gew.-%, vorzugsweise 0,05 bis 0,12 Gew.-%, in zwei Anteilen am Beginn der Anlagerungsreaktion und vor der Destillationsphase zugegeben. Die Anteile betragen mindestens 30 Gew.-%, vorzugsweise je 50 Gew.-%, bezogen auf die gesamte Menge der zugegebenen Oxalsäure.

Unter den phenolischen Antioxydantien hat sich 2,6-Di-tert.butyl-4-methylphenol als sehr günstig erwiesen. Es wird in Mengen von 0,06 bis zu 0,10 Gew.-%, bezogen auf den gesamten Reaktionsansatz, mitverwendet.

Die folgenden Beispiele erläutern die Erfindung, ohne diese in ihrem Umfang zu beschränken. Alle Angaben in Teilen oder Prozenten beziehen sich, soferne nicht anders angegeben, auf Gewichtseinheiten.

Beispiele 1 - 3:

In einer Universalkunstharzanlage werden das $C_8$-$C_{13}$-Monoolefin handelsüblicher technischer Qualität

oder Mischungen mehrerer $C_8$-$C_{18}$-Monoolefine, 2,6-Di-tert.butyl-4-methylphenol (DTBMP) gelöst in der dreifachen Menge des $C_8$-$C_{18}$-Monoolefins, und die Hälfte der insgesamt benötigten Oxalsäure (OS I) unter Inertgas auf 100 - 120°C erwärmt. Nach Zugabe des Maleinsäureanhydrids (MSA) wird die Temperatur auf 185 - 200°C erhöht und während 10 - 14 Stunden gehalten, bis das Maleinsäureanhydrid einen Restwert von 8 - 12 % bezogen auf das eingesetzte Maleinsäureanyhdrid erreicht hat. Nach dem Abkühlen auf 120°C wird dem Ansatz die zweite Hälfte der Oxalsäure (OS II) zugegeben. Anschließend werden nicht umgesetztes $C_8$-$C_{18}$-Monoolefin und Maleinsäureanhydrid unter Vakuum abdestilliert, wobei die Tempertur allmählich auf 185 - 210°C gesteigert wird. Beendet wird die Destillation, sobald der Ansatz einen Festkörpergehalt von über 98 % nach DIN 53 216-A erreicht hat.

Vergleichsbeispiel:

Wie beim erfindungsgemäßen Verfahren werden das $C_8$-$C_{18}$-Monoolefin, DTBMP und Triphenylphosphit TPP (als 50%ige Lösung in Xylol) unter Inertgas auf 100 - 120°C erwärmt. Nach Zugabe von MSA wird die Temperatur auf 185 - 200°C erhöht und während 10 - 14 Stunden gehalten, bis das Maleinsäureanhydrid einen Restwert von 8 - 12 %, bezogen auf das eingesetzte Maleinsäureanyhdrid, erreicht hat. Nach dem Abkühlen auf 150°C werden nicht umgesetztes $C_8$-$C_{18}$-Monoolefin und Maleinsäureanhydrid vorsichtig unter Vakuum abdestilliert, wobei die Temperatur allmählich auf 185 - 210°C gesteigert wird. Beendet wird die Destillation, sobald der Ansatz einen Festkörpergehalt von über 98 % nach DIN 53 216-A erreicht hat.

Die Mengenverhältnisse sowie die bei den einzelnen Beispielen erzielten Farbzahlen werden in der Tabelle 1 zusammengefaßt.

Tabelle 1

| | Beispiel 1 | Beispiel 2 | Beispiel 3 | Vergleichsbeispiel |
|---|---|---|---|---|
| Octen-1 | 370 | -- | -- | -- |
| | (3,3 Mol) | | | |
| Undecen-1 | -- | 447 | -- | 462 |
| | | (2,9 Mol) | | (3 Mol) |
| Tetradecen-1 | -- | -- | 491 | -- |
| | | | (2,5 Mol) | |
| DTBMP | 0,3 | 0,35 | 0,4 | 0,9 |
| TPP | -- | -- | -- | 1,1 |
| OS I | 0,15 | 0,2 | 0,3 | -- |
| MSA | 98 | 98 | 98 | 98 |
| | (1 Mol) | (1 Mol) | (1 Mol) | (1 Mol) |
| OS II | 0,15 | 0,2 | 0,3 | -- |
| Farbzahl nach DIN 6162 | 6 | 6 | 7 | 12 |

**Ansprüche**

1. Verfahren zur Herstellung von Alkenylbernsteinsäureanhydriden mit verbesserter Eigenfarbe durch Umsetzung von Maleinsäureanhydrid mit einem molaren Überschuß an $C_8$-$C_{18}$-Monoolefinen in Gegenwart eines phenolischen Antioxidans, dadurch gekennzeichnet, daß die Additionsreaktion in Gegenwart von, bezogen auf den gesamten Reaktionsansatz, 0,04 bis 0,15 Gew.-%, vorzugsweise 0,05 bis 0,12 Gew.-%, Oxalsäure und von 0,04 bis 0,12 Gew.-% eines phenolischen Antioxidans durchgeführt wird, mit der Maßgabe, daß die Oxalsäure in zwei Anteilen von mindestens 30 Gew.-%, bevorzugt in Anteilen von je 50 Gew.-%, bezogen auf die gesamte Menge an Oxalsäure zu Beginn der Additionsreaktion und vor der Destillationsphase zugegeben wird.

2. Verfahren nach Anspruch 1, dadurch gekennzeichnet, daß als phenolisches Antioxidans 2,6-Di-tert.butyl-4-methylphenol in Mengen von 0,06 bis 0,10 Gew.-%, bezogen auf den gesamten Reaktionsansatz, eingesetzt wird.

4